# FASCICULE DE BREVET EUROPEEN

(11) **EP 3 595 754 B1**
(45) Date de publication et mention de la délivrance du brevet: **26.10.2022**
(21) Numéro de dépôt: 18710067.2
(22) Date de dépôt: 16.03.2018
(51) Int. Cl.: A61M 5/32, A61M 5/31

(54) **DISPOSITIF DE PROTECTION D'AIGUILLE POUR UNE SERINGUE PRÉ-REMPLIE À AIGUILLE COLLÉE ET SERINGUE COMPRENANT UN TEL DISPOSITIF**
NADELSCHUTZVORRICHTUNG FÜR EINE VORGEFÜLLTE SPRITZE MIT ABGESTECKTER NADEL UND SPRITZE MIT SOLCH EINER VORRICHTUNG
NEEDLE PROTECTION DEVICE FOR A PRE-FILLED SYRINGE WITH STAKED NEEDLE, AND SYRINGE COMPRISING SUCH A DEVICE

(30) Priorité: 17.03.2017 FR 1752182
(43) Date de publication de la demande: 22.01.2020
(73) Titulaire: Biocorp Production, 63500 Issoire (FR)
(72) Inventeur: ANEAS, Daniel, 63200 Menetrol (FR)
(74) Mandataire: Lavoix
(86) Numéro de dépôt international: PCT/EP2018/056648
(87) Numéro de publication internationale: WO 2018/167262

(56) Documents cités:
- EP-A1- 3 106 194
- WO-A1-2016/158627
- WO-A2-2016/120185
- FR-A1- 3 031 904

## Description

L'invention concerne un dispositif de protection d'aiguille pour une seringue du type pré-remplie à aiguille collée. L'invention concerne également une seringue comprenant un tel dispositif.

Une seringue pré-remplie à aiguille collée est une seringue à usage unique, comprenant une aiguille hypodermique en métal, qui est collée à l'intérieur d'un canal défini dans le corps de seringue. La seringue est pré-remplie, dans le sens où l'infirmier (ou l'infirmière) n'a pas besoin de remplir la seringue, par exemple à partir d'un flacon, avant d'effectuer la piqûre.

De manière connue en soi dans le domaine médical, les seringues à aiguille collée sont équipées d'un protège-aiguille, à l'intérieur duquel l'aiguille est enfoncée. Souvent, le protège-aiguille est en caoutchouc ou en élastomère. Il existe des protège-aiguilles dits « rigides », comprenant un corps interne en matériau souple et un corps externe en matériau rigide. Le corps interne assure l'étanchéité du principe actif contenu à l'intérieur de la seringue par rapport à l'extérieur et le corps externe protège l'aiguille des chocs. Notamment, le corps externe peut empêcher l'aiguille de se tordre.

Les seringues à aiguille collée doivent de préférence intégrer un système de sécurité après usage. Ce système de sécurité après usage a pour objectif d'éviter tout contact avec l'aiguille une fois que l'injection est terminée. Il vise donc à limiter le risque de contamination par l'aiguille et donc la transmission de maladies, comme le VIH. Pour information, un tel système est obligatoire d'un point de vue légal dans des pays comme les Etats-Unis et le Canada.

A cet effet, la société déposante, BIOCORP PRODUCTION, a développé un dispositif de protection spécifique en ce qu'il comprend d'une part, un protège-aiguille rigide (« rigid needle shield » en anglais) composé d'une partie souple enveloppée dans une gaine rigide et, d'autre part, un système de sécurité après usage (« safety device » en anglais) composé d'un fourreau et d'un ressort. Ce dispositif de protection est décrit notamment dans les demandes internationales WO 2016/120185 A1 et WO 2016/207196 A1.

Ce dispositif est perfectible en plusieurs points.

Le premier point concerne la fabrication du fourreau. Celui-ci présentait des défauts de cylindricité provenant du fait que le procédé d'injection plastique utilisait un point d'injection désaxé par rapport à l'axe central du fourreau. Effectivement, du fait de la géométrie creuse et tubulaire du fourreau, il n'était pas possible d'injecter la matière par le centre.

Le deuxième point concerne la mise en place du protège-aiguille sur l'extrémité du corps de seringue. Effectivement, lors du montage du dispositif, la partie souple du protège-aiguille était comprimée dans le sens de la longueur de façon à assurer l'étanchéité avec le corps de seringue. La compression était effectuée en appuyant à l'extrémité de la partie souple par l'intermédiaire d'un ergot formé à l'intérieur de la gaine rigide du protège-aiguille. Cette surface d'appui n'était pas forcément adaptée, dans le sens où cela pouvait entrainer des défauts de montage. Notamment, la partie souple du protège-aiguille pouvait vriller pendant le montage.

Le troisième point concerne le fait que, avec ce dispositif, rien n'empêchait de reculer manuellement le fourreau. Le fourreau pouvait donc être déplacé en configuration de fin d'injection, configuration dans laquelle il était verrouillé. Dans un tel cas de figure, l'aiguille ne peut pas être découverte et la seringue est inutilisable, ce qui n'est évidemment pas souhaitable. Un tel déplacement pouvait intervenir pendant la mise en place à l'intérieur des logements définis dans les supports standards utilisés pour le transport et le conditionnement (mieux connus sous le nom de « nest » en anglais), pendant les phases de convoyage, d'emballage, de transport ou encore avant l'utilisation.

Le quatrième point concerne le fait que les utilisateurs avaient du mal à comprendre quelle était la partie détachable et comment la détacher.

Le cinquième point concerne le phénomène de « coring », ou carottage en français. Ce phénomène intervient lors d'un mouvement relatif entre l'aiguille et le protège-aiguille : la forme biseautée de la pointe de l'aiguille a tendance à « creuser » la matière constitutive du protège-aiguille et générer des copeaux de matière élastomère susceptibles de tomber à l'intérieur de l'aiguille.

Enfin, le sixième point concerne l'étape de stérilisation des seringues. De manière connue en soi, les seringues, équipées du dispositif de protection, sont stérilisées avec un gaz spécifique, notamment avec de l'ETO (oxyde d'éthylène). Ensuite, certaines seringues pré-remplies sont stérilisées, avec leur contenu, dans un autoclave, par exemple avec de la vapeur d'eau. Lors de cette étape, l'augmentation de pression à l'intérieur de la chambre intérieure de la partie souple du protège-aiguille pouvait entraîner un décollement du protège-aiguille et ce décollement était susceptible de conduire à un défaut d'étanchéité avec le corps de seringue. Ce phénomène porte un nom : il s'agit du « pop-off ». Egalement, le même effet pouvait être obtenu lors d'un choc, de vibrations (par exemple pendant le transport) conduisant au glissement du protège-aiguille.

Ainsi, l'invention entend proposer un dispositif de protection d'aiguille amélioré.

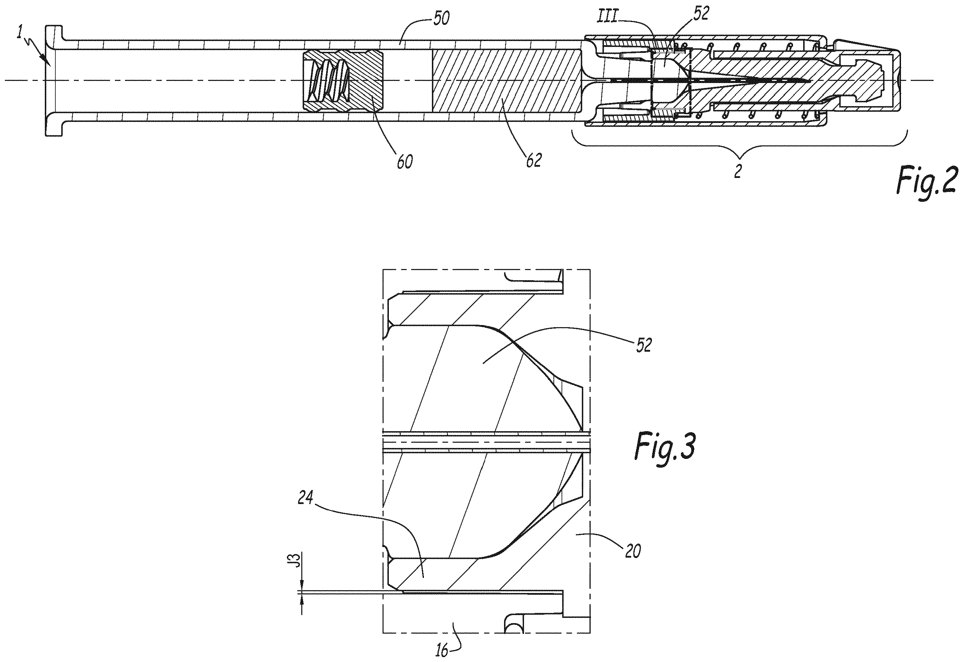

WO 2016/158 627 A1, qui peut être considéré comme l'état de la technique le plus proche, divulgue un dispositif de protection d'aiguille, dans lequel le corps extérieur du protège-aiguille (rigide) est fixé de façon amovible avec le fourreau. Notamment, le corps extérieur délimite un épaulement radial de réception d'un rebord périphérique intérieur formé à une extrémité axiale du fourreau. Dans le paragraphe [0051] de WO 2016/158 627 A1, il est indiqué que le tube interne 48 est fait d'un matériau dont la rigidité est supérieure à celle du protège-aiguille 46, par exemple une résine. Par conséquent, le tube interne 48 est monté à l'extérieur du protège-aiguille 46 de telle sorte que le corps du protection d'aiguille 34 est configuré de manière à présenter une rigidité suffisante. Ainsi, l'utilisateur peut facilement pousser le protège-aiguille 46 dans le tube extérieur 32 par l'intermédiaire du tube interne 48 ou détacher l'ensemble protège-aiguille 46 plus tube interne 48 de l'aiguille 16. La teneur de ce paragraphe [0051], ainsi que la représentation de la figure 3 par exemple, indiquent clairement que le tube interne 48 et le tube extérieur 32 ne sont pas fabriqués en une seule pièce par injection plastique.

WO 2014/131985 A1 divulgue un dispositif de protection d'aiguille comprenant un protège-aiguille rigide, dans lequel le corps externe est fixé de façon amovible au bloqueur par l'intermédiaire de pontets sécables et dans lequel le corps externe et le corps interne du protège-aiguille sont liés axialement en translation. Le bord d'extrémité axiale du corps externe est rabattu vers l'intérieur pour maintenir fixement le corps interne.

FR 3 031 904 A1 divulgue un dispositif de protection d'aiguille comprenant un protège-aiguille rigide, dans lequel le corps externe est fixé de façon amovible au bloqueur par l'intermédiaire de pontets sécables et dans lequel le corps externe et le corps interne du protège-aiguille sont liés axialement en translation. Le bord d'extrémité axiale du corps externe est rabattu vers l'intérieur pour maintenir fixement le corps interne.

EP 3 106 194 A1 divulgue un dispositif de protection d'aiguille, dans lequel un capuchon est fixé de façon amovible au fourreau et est réalisé d'une seule pièce avec le protège-aiguille. Un bloqueur est immobilisé sur le corps de seringue et comprend deux pions radiaux insérés chacun dans une ouverture radiale de guidage du fourreau.

WO 2013/134465 A1 divulgue un dispositif de protection d'aiguille comprenant un système de sécurité après usage et un protège-aiguille souple, à l'intérieur duquel est enfoncée l'aiguille. Le système de sécurité après usage comprend un fourreau extérieur et un ressort. Un bloqueur est immobilisé sur le corps de seringue. Ce bloqueur comprend deux pions radiaux insérés chacun dans une ouverture radiale de guidage du fourreau.

US 2015/0246182 A1 divulgue un dispositif de protection d'aiguille, dans lequel un capuchon est fixé de façon amovible au fourreau et est lié axialement avec le protège-aiguille (souple). Notamment, le capuchon comprend deux boutons poussoirs servant à pincer le protège-aiguille pour le retirer. Le nombre de pièces utilisées et leur complexité rendent ce dispositif cher à fabriquer, et donc peu intéressant d'un point de vue industriel. De plus, si l'utilisateur tourne (même légèrement) le capuchon pendant le retrait du protège-aiguille, il y a un risque de carottage.

WO 2015/022 787 A1 divulgue un dispositif de protection d'aiguille, dans lequel le protège-aiguille (souple) est lié de façon détachable avec le fourreau. Notamment, le protège-aiguille délimite une gorge périphérique à l'intérieur de laquelle est reçue une nervure périphérique intérieure du fourreau, laquelle est formée à une extrémité axiale de ce dernier.

WO 2016/206 862 A1 divulgue un exemple de système de sécurité après usage pour une seringue à aiguille collée. Ce système comprend, de manière connue en soi, un fourreau extérieur de protection et un ressort agencé pour charger élastiquement le fourreau vers l'avant, c'est-à-dire de façon distale par rapport au corps de seringue. Le fourreau délimite une ouverture radiale de guidage d'un pion formé sur un bloqueur monté fixement à une extrémité du corps de seringue.

WO 2016/202 614 A1 divulgue un dispositif de protection d'aiguille dans lequel le protège-aiguille est prévu sous la forme d'un capuchon en matériau souple. Ce capuchon comprend une partie extérieure configurée pour recouvrir radialement le fourreau et une partie coaxiale interne à l'intérieur duquel l'aiguille est enfoncée. Le capuchon est fixé de manière amovible au fourreau grâce à un système de clipsage. WO 2016/202 510 A1 a sensiblement le même enseignement technique.

WO 2016/202 498 A1 diffère des deux précédents documents, dans le sens où le protège-aiguille est bipartite. Il comprend une partie intérieure souple à l'intérieur de laquelle l'aiguille peut être enfoncée et une partie extérieure agencée coaxialement autour du fourreau. La partie extérieure est liée axialement en translation avec la partie intérieure et est clipsée avec le fourreau.

À cet effet, l'invention concerne un dispositif de protection d'aiguille selon la revendication 1.

Aucun des documents d'art antérieur ne divulgue un fourreau de protection comprenant un tube intérieur et un tube extérieur fabriqués en une seule pièce par injection plastique (moulage par injection). Le dispositif de protection d'aiguille selon l'invention est donc plus simple et moins cher à fabriquer que les dispositifs de l'art antérieur. En outre, le tube intérieur du fourreau s'oppose au déplacement du tube externe vers l'arrière, c'est-à-dire en direction du corps de seringue. Ainsi, le fourreau est immobilisé en translation tant que la partie amovible de ce dernier, à savoir le tube interne, est encore en place. Ainsi, il n'y aucun risque que le tube externe du fourreau se retrouve en configuration de fin d'injection par erreur, c'est-à-dire suite à une fausse manipulation ou à un choc.

Selon des aspects avantageux mais non obligatoires de l'invention, un tel dispositif peut incorporer une ou plusieurs des caractéristiques suivantes, prises dans toute combinaison techniquement admissible :
- Le protège-aiguille comprend une collerette interposée axialement entre le bloqueur et le tube interne du fourreau.

De cette manière, la collerette forme une butée axiale au tube interne du fourreau et s'oppose au recul du fourreau tant que le tube interne n'a pas été détaché. Egalement, le tube interne du fourreau s'oppose, au jeu axial près, au glissement du protège-aiguille vers l'avant autour du corps de seringue, par exemple sous l'effet de vibrations occasionnées pendant le transport. De plus, il n'y a aucun risque de « pop-off » pendant l'opération de stérilisation dans l'autoclave, lorsque celle-ci est effectuée, dans la mesure où le tube interne du fourreau s'oppose au décollement du protège-aiguille, c'est-à-dire au déplacement du protège-aiguille selon une direction distale par rapport au corps de seringue. Egalement, lorsque l'on appuie sur le tube interne lors du montage, l'effort est principalement transmis par le tube interne du fourreau, puis par la collerette. La part du protège-aiguille dans la transmission d'efforts est donc minoritaire, de façon que le risque que le protège-aiguille vrille ou se mette de travers est réduit, voire supprimé.
- Le tube externe et le tube interne sont attachés l'un à l'autre par des pontets sécables.

Les pontets sont configurés pour être rompus lorsqu'un couple est appliqué, sur le tube interne du fourreau, autour de l'axe central de celui-ci et que le tube externe est manuellement retenu, c'est-à-dire immobilisé en rotation. En d'autres termes, les pontets sont cassés en imposant une rotation relative entre le tube interne et le tube externe. Le tube interne est libre en rotation (autour de son axe) par rapport au protège-aiguille. Le tube interne peut donc être pivoté autour de son axe sans faire tourner le protège-aiguille. Le fourreau, et plus particulièrement le tube interne, a une fonction de protection mécanique de l'aiguille. Il empêche celle-ci de se tordre.
- Les pontets sécables sont formés à une extrémité axiale du tube externe.

Contrairement au dispositif de WO 2016/120185 A1 et WO 2016/207196 A1, les pontets sont disposés à l'extérieur du tube externe, si bien que l'effort à appliquer pour rompre les pontets est moindre.
- Le tube interne comprend une extrémité fermée.

Etant donné que le tube externe et le tube interne sont une même pièce, le fait d'avoir cette extrémité fermée a pour avantage que le point d'injection de matière pendant le procédé d'injection plastique peut être prévu sur l'axe central du fourreau, ce qui permet d'obtenir une répartition de matière homogène, sans défaut de cylindricité et d'éviter toute ligne de soudure. En outre, cela permet de couvrir totalement le protège-aiguille. Ainsi, le protège-aiguille est complètement isolé de l'extérieur, et notamment des poussières. Ceci est plus agréable esthétiquement pour les utilisateurs, c'est-à-dire le personnel médical.
- Le tube interne présente un diamètre extérieur minimum strictement inférieur à un diamètre extérieur d'une partie d'extrémité distale du bloqueur.

Grâce à cette caractéristique, le montage du protège-aiguille autour de l'extrémité du corps de seringue est facilité. Effectivement, nous avons indiqué plus haut que la collerette du protège-aiguille était interposée axialement entre le tube interne et le bloqueur. Le protège-aiguille comporte une jupe destinée à épouser la forme de l'extrémité du corps de seringue. Lors du montage du protège-aiguille, la collerette est donc comprimée entre le bloqueur et le tube interne. La différence de diamètre susmentionnée a pour effet que les efforts appliqués respectivement par le bloqueur et le tube interne sont désaxés l'un par rapport à l'autre, ce qui génère en fait un couple dirigé vers l'extérieur, c'est-à-dire que ce couple a tendance à ouvrir la jupe du protège-aiguille. Autrement dit, les efforts résultants exercés par le tube interne sur la collerette sont plus éloignés de l'axe central que les efforts résultants exercés par le bloqueur sur la collerette. Le couple résultant favorise l'emmanchement de la jupe du protège-aiguille autour de l'extrémité du corps de seringue et empêche le protège-aiguille de s'affaisser contre l'extrémité du corps de seringue.
- Le tube interne est opaque et le tube externe est transparent.

Cette caractéristique permet de répondre à un besoin utilisateur suivant lequel la partie détachable du dispositif doit être clairement identifiable et suivant lequel l'aiguille doit rester visible une fois le protège-aiguille retiré.
- Le tube interne et le tube externe présentent un état de surface différent.

Cet état de surface différencié permet de créer l'illusion de deux pièces distinctes, même si le tube interne et le tube externe sont en fait une même pièce fabriquée par injection plastique. Avantageusement, la matière plastique utilisée pendant l'injection est de base sensiblement transparente, ou au moins translucide. Les surfaces du moule utilisé pour la fabrication du fourreau sont réalisées de façon à obtenir un état de surface (rugosité) différent entre le tube interne et le tube externe. En particulier, le tube externe présente des surfaces très lisses, grâce à un procédé communément appelé poli-glace, ce qui lui donne un aspect très transparent. En revanche, le tube interne présente des surfaces plus rugueuses, c'est-à-dire un état de surface plus grossier, ou plus brut, que l'on appelle communément « grains charmille », et qui confère un aspect opaque.
- Le tube interne comprend des ailettes de préhension.

Ces ailettes de préhension forment un indice supplémentaire pour l'utilisateur sur quelle partie du dispositif est détachable. Egalement, les ailettes permettent de définir un tronçon de préhension de plus grand diamètre (par rapport au reste du tube), ce qui fait que l'effort à fournir pour rompre les pontets sécables est nécessairement moins important, du fait de l'augmentation du bras de levier.
- Le bloqueur comprend au moins un pion engagé dans une ouverture radiale de guidage du premier tube.

L'invention concerne également une seringue pré-remplie à aiguille collée, comprenant un dispositif tel que décrit précédemment.

De préférence, le bloqueur est monté libre en rotation autour d'un corps de seringue.

Avantageusement, le bloqueur est monté avec un jeu radial autour du protège-aiguille.

Ainsi, le bloqueur peut tourner librement en configuration montée du dispositif sur le corps de seringue. L'ensemble bloqueur plus fourreau est donc complètement libre en rotation autour du corps de seringue et autour du protège-aiguille. En rendant le bloqueur complètement libre en rotation autour du protège-aiguille, on supprime totalement le risque de carottage pendant toutes les phases d'utilisation de la seringue.

Lors d'une piqûre, le fourreau est immobilisé en rotation au contact de la peau. En revanche, le bloqueur peut s'orienter par rapport au fourreau pour assurer le guidage des pions dans les ouvertures radiales correspondantes du fourreau. Egalement, puisqu'il n'y a pas de rotation relative du fourreau par rapport au bloqueur, il est impossible, en fin d'injection, de réarmer le fourreau en position de recouvrement de l'aiguille.

L'invention et d'autres avantages de celle-ci apparaitront plus clairement à la lumière de la description qui va suivre d'un mode de réalisation d'un dispositif de protection d'aiguille, donnée uniquement à titre d'exemple et faite en référence aux dessins dans lesquels :
- la figure 1 est une vue en perspective éclatée d'un dispositif de protection d'aiguille conforme à l'invention, destiné à être monté sur une seringue pré-remplie à aiguille collée ;
- la figure 2 est une vue en coupe du dispositif de la figure 1 en configuration assemblée sur un corps de seringue,
- la figure 3 est un détail de l'encadré III de la figure 2,
- la figure 4 est une vue en coupe du dispositif des figures 1 et 2 représenté seul, et
- les figures 5 à 8 sont des vues en coupe représentant successivement des étapes de montage du dispositif des figures 1 à 3 sur un corps d'une seringue à aiguille collée.

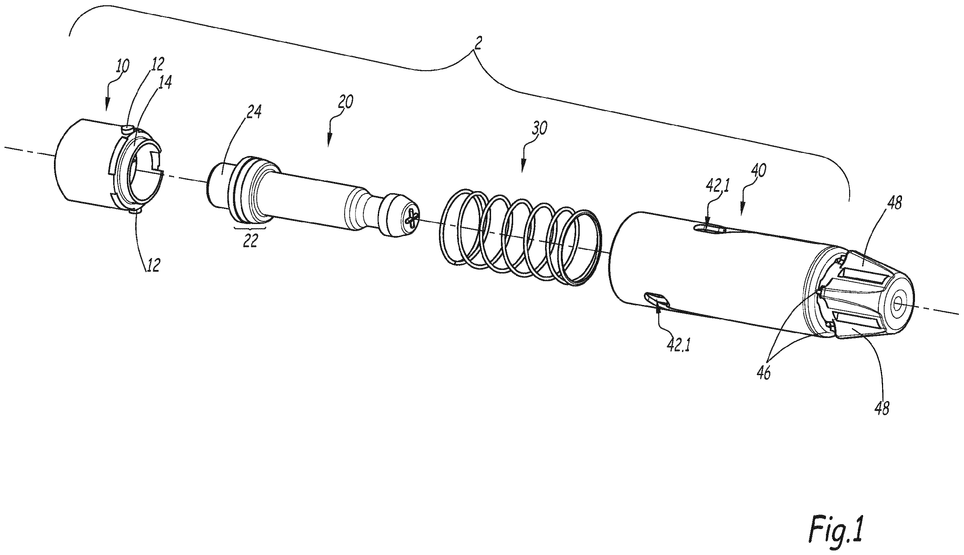

Sur les figures 1 à 4 est représenté un dispositif 2 de protection d'aiguille. Comme visible à la figure 2, le dispositif 2 est configuré pour être monté sur un corps 50 de seringue 1, notamment à une extrémité 52 du corps de seringue 50, que l'on appelle couramment le nez de la seringue. La seringue 1 est une seringue pré-remplie à aiguille collée. Elle comprend un piston 60 (ou joint de piston) vissé à une tige de piston (non représentée). Un principe actif 62 est contenu à l'intérieur du corps de seringue 50. La partie d'extrémité 52 du corps de seringue 50 délimite un canal 54 à l'intérieur duquel est collé une aiguille hypodermique 58. L'aiguille 58 est en métal et présente une extrémité biseautée.

Avantageusement, le corps de seringue 50 est standard, c'est-à-dire qu'il s'agit du corps de seringue vendu en majorité, au moins en Europe, pour ce type d'application. Pour information, ce type de corps de seringue représente la majorité des parts de marché en Europe.

Le dispositif de protection d'aiguille 2 définit un axe central X2. Il comprend un bloqueur 10, un protège-aiguille 20, un ressort 30 et un fourreau 40.

Dans le présent document, on définit une direction proximale (ou arrière) comme une direction parallèle à l'axe central X2 et orientée vers le corps de seringue 50. A l'inverse, une direction distale (ou avant) désigne une direction parallèle à l'axe X2 mais orientée à l'opposé par rapport au corps de seringue 50.

Egalement, l'adjectif « radial » se rapporte à une surface perpendiculaire à l'axe X2, alors que l'adjectif « axial » se rapporte à une surface parallèle à l'axe X2.

Le bloqueur 10, qui peut également être connu sous le nom de collier (« collar » en anglais), présente une géométrie sensiblement révolutive. Il comprend des moyens 16 de fixation sur le corps de seringue 50. Ces moyens de fixation 16 comprennent des pattes élastiques, qui sont dans l'exemple au nombre de trois et qui sont configurées pour venir s'encliqueter, c'est-à-dire se « clipser », autour de la partie d'extrémité 52 du corps de seringue 50. A cet effet, la partie d'extrémité 52 délimite un col 56 contre lequel les pattes élastiques 16 peuvent venir se fixer. Avantageusement, le bloqueur 10 comprend au moins un, de préférence deux pions radiaux 12 qui font saillie radialement vers l'extérieur. Dans l'exemple, les pions 12 sont diamétralement opposés.

Le bloqueur 10 comprend un épaulement définissant une partie d'extrémité distale 14, dont le diamètre extérieur D14 est réduit par rapport au reste du bloqueur.

Le bloqueur 10 du dispositif de protection d'aiguille 2 est plus facile à fabriquer que le collier du dispositif de WO 2016/120185 A1. Effectivement, dans cette publication, le collier était fabriqué en une seule pièce avec une gaine rigide entourant le protège-aiguille. Or, lorsque l'on retirait le noyau utilisé pour le moulage, les reliefs intérieurs du collier formaient une contre-dépouille qui s'opposait à ce retrait, ce qui entrainait des arrachements de matière. A l'inverse, le bloqueur 10 est fabriqué suivant un procédé d'injection plastique utilisant deux noyaux séparés, lesquels sont retirés chacun d'un côté du bloqueur, de façon à éviter tout arrachement de matière. Cela est rendu possible car le tube 44 et le bloqueur 10 ne sont pas attachés l'un à l'autre, du moins pas de façon directe comme dans WO 2016/120185 A1.

Les pions 12 du bloqueur 10 sont avantageusement engagés dans des ouvertures radiales correspondantes 42.1 du fourreau 40. Les ouvertures 42.1 sont sensiblement en forme de « Y ». Elles sont identiques à celles décrites dans les publications WO 2016/120185 A1 et WO 2016/207196 A1 c'est pourquoi elles ne sont pas décrites plus avant. Le fourreau 40 est lié en rotation avec le bloqueur 10 autour de l'axe central X2 du fait de la coopération entre les pions 12 et les ouvertures 42.1. En outre, la coopération entre les pions 12 et les ouvertures 42.1 du fourreau empêche de déplacer le fourreau 40 dans la direction distale par rapport au bloqueur 10, notamment car, en configuration de stockage, les pions 12 sont en butée dans les ouvertures 42.1.

Avantageusement, les ouvertures 42.1 servent de passage aux gaz de stérilisation, tel que l'oxyde d'éthylène, lors de la stérilisation de la seringue.

Le fourreau 40 comprend un tube externe 42 délimitant les ouvertures 42.1 et un tube interne 44. Le tube externe 42 et le tube interne 44 sont fabriqués en une seule pièce par injection plastique, ou moulage par injection. Le tube interne 44 est disposé coaxialement à l'intérieur du tube externe 42 et dépasse axialement, côté distal, par rapport au tube externe 42.

Astucieusement, le tube interne 44 du fourreau 40 s'oppose au décollement du protège-aiguille 20, c'est-à-dire au déplacement du protège-aiguille 20 selon une direction distale par rapport au corps de seringue 50, pendant l'opération de stérilisation en autoclave. Egalement, le tube interne 44 s'oppose au glissement du protège-aiguille 20 autour de l'extrémité 52 du corps de seringue 50, du fait des vibrations occasionnées pendant le transport ou autre.

Le fourreau 40, et plus particulièrement le tube interne 44, a également pour fonction de protéger l'aiguille 58, et notamment d'empêcher que l'aiguille se torde. Il peut être assimilé à une partie rigide de protège-aiguille (« Rigid Needle Shield » en anglais).

Le tube externe 42 et le tube interne 44 sont attachés l'un à l'autre par des pontets sécables 46. Dans l'exemple, les pontets 46, ou picots, sont au nombre de quatre.

Avantageusement, les pontets 46 sont configurés pour être rompus lorsqu'un couple est appliqué, sur le tube interne 44 du fourreau 40, autour de l'axe central de celui-ci et que le tube externe 42 est retenu manuellement, c'est-à-dire immobilisé en rotation. Le tube interne 44 est libre en rotation (autour de son axe) par rapport au protège-aiguille 20. Le tube interne peut donc être pivoté autour de son axe sans faire tourner le protège-aiguille 20. Effectivement, il est impératif de ne pas créer de mouvement de rotation du protège-aiguille 20 autour de l'aiguille 58 car la forme biseautée de la pointe de l'aiguille aurait tendance à « creuser » la matière constitutive du protège-aiguille et générer des copeaux de matière susceptibles de tomber à l'intérieur de l'aiguille (phénomène de « coring »).

Notamment, les pontets sécables 46 sont formés à une extrémité distale 42.2 du tube externe 42. Ils sont donc disposés à l'extérieur du volume interne du tube 42. Par rapport au dispositif de WO 2016/120185 A1 et WO 2016/207196 A1, l'effort à appliquer pour rompre les pontets 46 est donc moindre.

Avantageusement, l'extrémité distale 42.2 comprend un bord interne chanfreiné 42.20, évasé vers l'intérieur du tube 42.

De préférence, le tube interne 44 n'est pas traversant puisqu'il comprend avantageusement une paroi d'extrémité 44.2 obturant l'extrémité distale du tube 44. Grâce à cette paroi 44.2, le point d'injection 44.3 de matière pendant l'injection plastique peut être positionné au niveau de l'axe central, ou de révolution, du fourreau 40. Cela assure une répartition de matière homogène dans le moule et des tolérances de cylindricité très faibles. En outre, étant donné que l'on utilise un seul point d'injection centralisé, il n'y a pas de ligne de soudure. Classiquement, les lignes de soudure sont les lignes au niveau desquelles se rejoint la matière injectée dans le moule. Le fait de ne pas avoir de lignes de soudure est particulièrement avantageux puisque les lignes de soudure (ou de jonction) forment traditionnellement des points de faiblesse mécanique. Egalement, cette paroi 44.2 permet de recouvrir totalement le protège-aiguille 20 et donc protège des poussières, ce qui est plus esthétique, mais important dans le milieu hospitalier.

On note D44 le diamètre extérieur minimum du tube interne 44. Ce diamètre peut notamment être mesuré au niveau de l'extrémité proximale du tube 44.

Avantageusement, le tube interne 44 comporte un moyen de rétention axiale, formé dans l'exemple par au moins une, de préférence plusieurs nervures internes 44.1 qui s'étendent, par secteur, selon une direction périphérique (ortho-radiale) autour de l'axe central du dispositif.

Des ailettes 48 sont formées sur la partie visible du tube interne 44, c'est-à-dire la partie du tube interne 44 disposée à l'extérieur du tube externe 42. Ces ailettes 48 sont des ailettes de préhension invitant l'utilisateur à manipuler le tube interne 44 pour le détacher du tube externe 42. Une surface d'enveloppe des ailettes 48 (non représentée) présente un diamètre inférieur ou égal au diamètre extérieur du tube externe 42 du fourreau 40. De cette façon, les ailettes 48 ne sont pas un obstacle à la mise en place (et au retrait) des seringues à l'intérieur des « nest ».

Le protège-aiguille 20 est disposé avec un jeu radial à l'intérieur du tube interne 44 du fourreau 40, de façon que le tube 44 peut tourner librement autour du protège-aiguille 20. Le protège-aiguille 20 est réalisé dans un matériau souple, tel qu'un élastomère, notamment un élastomère thermoplastique (« TPE »), ou un caoutchouc. Notamment, le matériau constitutif du protège-aiguille 20 est tel que l'aiguille 58 de la seringue peut être enfoncée à l'intérieur de ce dernier. Egalement, ce matériau est perméable aux gaz, de façon que l'oxyde d'éthylène (ETO) peut pénétrer à l'intérieur de la jupe 24 pendant la stérilisation.

Le protège-aiguille 20 comprend, du côté proximal, une jupe 24 destinée à être emmanchée autour du nez 52 de la seringue et épouser ainsi la forme du nez 52.

Le protège-aiguille 20 comporte par ailleurs, côté distal, une partie à section réduite et un épaulement périphérique 26, élargissant le diamètre extérieur du protège-aiguille 20 vers le côté distal. L'épaulement 26 est configuré pour coopérer avec le moyen de rétention 44.1 du tube 44. Notamment, la nervure 44.1 est prévue pour venir en butée contre l'épaulement 26 lorsque le tube 44 est détaché axialement du tube 42. Ainsi, le protège-aiguille 20 et le tube interne 44 du fourreau 40 sont liés axialement en translation. Il est évident que les moyens de liaison entre le protège-aiguille 20 et le tube interne 44 ne sont pas limités à l'exemple de réalisation décrit et que d'autres solutions peuvent être mises en oeuvre pour assurer une liaison axiale entre le protège-aiguille 20 et le tube interne 44 du fourreau 40.

Le protège-aiguille 20 comporte par ailleurs une collerette 22 destinée à être interposée axialement entre le tube interne 44 et le bloqueur 10, notamment entre l'extrémité proximale du tube interne 44 et la partie d'extrémité distale 14 du bloqueur 10.

La collerette 22 comprend, côté distal, une pente 22.1, ou chanfrein, et, côté proximal, un bord radial 22.2. La collerette 22 forme avantageusement une butée axiale au tube interne 44 du fourreau 40 et s'oppose au recul du fourreau 40 tant que le tube interne 44 n'a pas été détaché. En pratique, il existe néanmoins, pour des raisons techniques, un jeu axial J entre l'extrémité du tube 44 et la collerette 22. Ce jeu J est astucieusement choisi inférieur à la longueur de piquage de l'aiguille 58, c'est-à-dire à la longueur sur laquelle l'aiguille 58 est enfoncée à l'intérieur du protège-aiguille 20. Par conséquent, il n'y a aucun risque que l'aiguille 58 sorte du protège-aiguille pendant l'opération de stérilisation dans l'autoclave. Dans l'exemple, le jeu J est de l'ordre de 0,4 mm.

Le ressort 30 est un ressort spiral s'étendant entre un épaulement du bloqueur 10 et l'extrémité distale 42.2 du tube externe 42, laquelle s'étend radialement vers l'intérieur du tube. Le ressort 30 forme un moyen pour charger élastiquement le tube externe 42 dans une position avancée où il est apte à recouvrir l'aiguille 58.

Avantageusement, le tube externe 42 est transparent, alors que le tube interne 44 est opaque. Cela donne l'illusion que le tube externe 42 et le tube interne 44 sont deux pièces différentes. Cela répond avantageusement à un besoin utilisateur suivant lequel la partie détachable du dispositif 2, c'est-à-dire le tube 44, doit être clairement identifiable et suivant lequel l'aiguille 58 doit rester visible une fois le protège-aiguille 20 retiré.

Dans l'exemple, l'illusion provient d'un traitement de surface distinct, dans le moule, entre le tube interne 44 et le tube externe 42. La matière plastique utilisée à la base dans le moule est sensiblement transparente, ou du moins translucide. Les surfaces du moule utilisé pour la fabrication du fourreau 40 sont réalisées de façon à obtenir un état de surface (rugosité) différent entre le tube interne 44 et le tube externe 42. En particulier, le tube externe 42 présente des surfaces très lisses, grâce à un procédé communément appelé poli-glace, ce qui lui donne un aspect très transparent. En revanche, le tube interne 44 présente des surfaces plus rugueuses, c'est-à-dire un état de surface plus grossier, ou plus brut, que l'on appelle communément « grains charmille », et qui confère un aspect opaque.

Dans l'exemple, et comme mentionné dans WO 2016/120185 A1, le dispositif 2 a un diamètre maximum (extérieur), qui est inférieur ou égal à 9,3 mm. Cela permet de rentrer les corps de seringue 50, avec leur dispositif 2, dans les logements d'un support (ou « nest ») standard, c'est-à-dire majoritaire sur le marché et dont les logements (trous) présentent un diamètre de 9,3 mm.

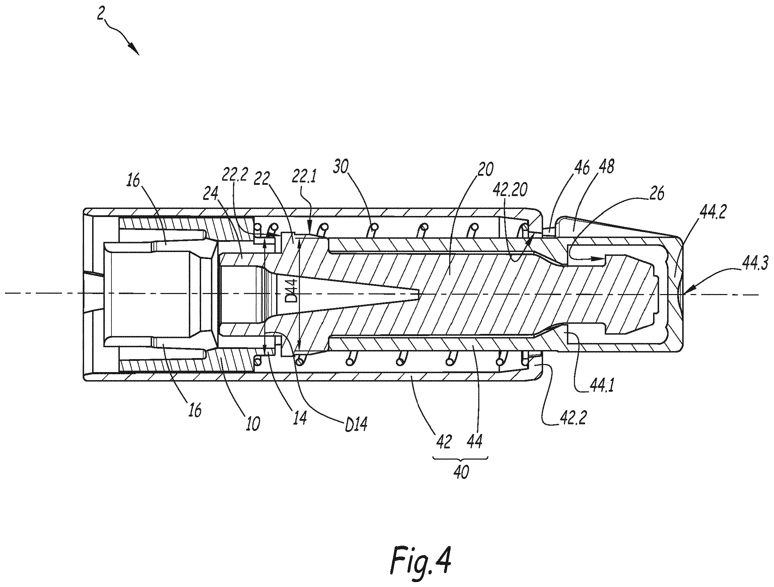

L'assemblage du dispositif 2 est effectué de la manière suivante. Le protège-aiguille 20 est monté à l'intérieur du fourreau 40. Pour cela, le protège-aiguille 20 est inséré par l'extrémité proximale du fourreau 40 à l'intérieur du tube interne 44. Le protège-aiguille 20 se comprime alors pour passer la nervure 44.1 du tube interne 44 et vient se positionner naturellement dans la position de la figure 4.

Vient ensuite le montage du ressort 30 et celui du bloqueur. Notamment, le bloqueur 10 est inséré à l'intérieur du fourreau 40, dans l'exemple en force. En variante, des rainures axiales pourront guider les pions 12 du bloqueur 10 jusqu'aux ouvertures de guidage 42.1. L'ordre dans lequel sont mises en œuvre les étapes de montage ci-dessus n'est pas important.

Le montage du dispositif 2 sur le corps de seringue 50 est effectué de la manière suivante. Le procédé de montage est identique, sinon équivalent au procédé actuel.

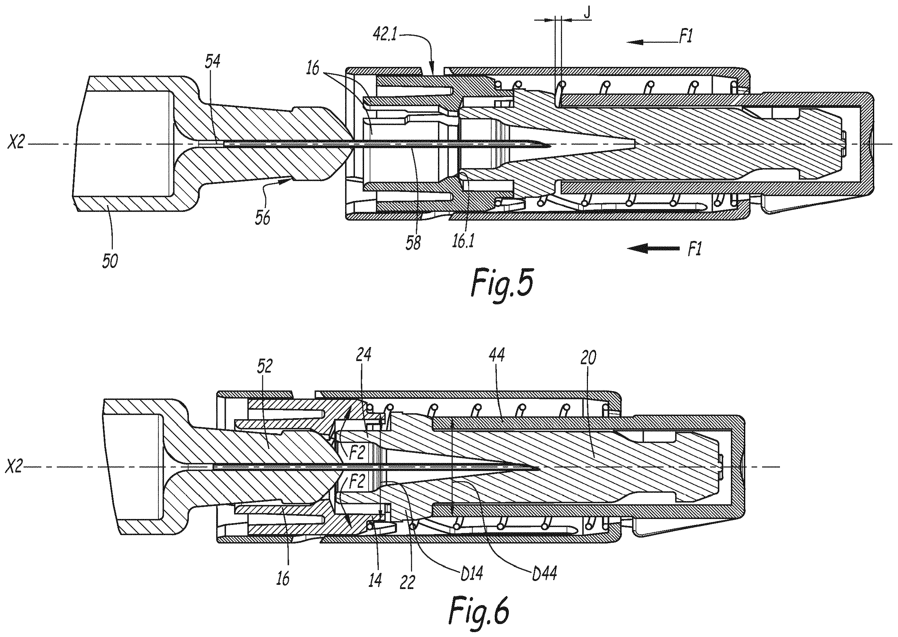

Le dispositif 2 est rapproché axialement de l'extrémité 52 du corps de seringue 50 de façon à enfoncer l'aiguille 58 à l'intérieur du protège-aiguille 20. Ce mouvement de rapprochement est représenté par les flèches F1 à la figure 5. Les pattes élastiques 16 viennent s'encliqueter autour de la partie d'extrémité 52, notamment grâce à la coopération entre l'épaulement 56 et les saillies 16.1 des pattes 16. En parallèle, la collerette 22 du protège-aiguille 20 est comprimée axialement entre le tube interne 44 du fourreau 40 et la partie d'extrémité distale 14 du bloqueur 10.

Étant donné que, lorsque l'on appuie sur le tube interne 44 lors du montage, l'effort est transmis au niveau de la collerette 22, le risque que le protège-aiguille 20 vrille ou se mette de travers pendant le montage est réduit, voire supprimé.

De plus, le diamètre D14 de la partie d'extrémité distale 14 du bloqueur 10 étant supérieur au diamètre D44 du tube interne 44, un couple est généré. Ce couple a tendance à écarter l'ouverture de la jupe 24 du protège-aiguille 20, comme représenté par les flèches F2 à la figure 6. La jupe 24 vient alors s'emmancher plus facilement autour de la partie d'extrémité 52 du corps de seringue 50.

Comme visible à la figure 3, il existe un jeu radial J3 entre le protège-aiguille 20 et le bloqueur 10. Le bloqueur 10 est donc libre de tourner autour du protège-aiguille 20, et notamment autour de la jupe 24. L'ensemble fourreau plus bloqueur est donc complètement libre en rotation autour du corps de seringue 50.

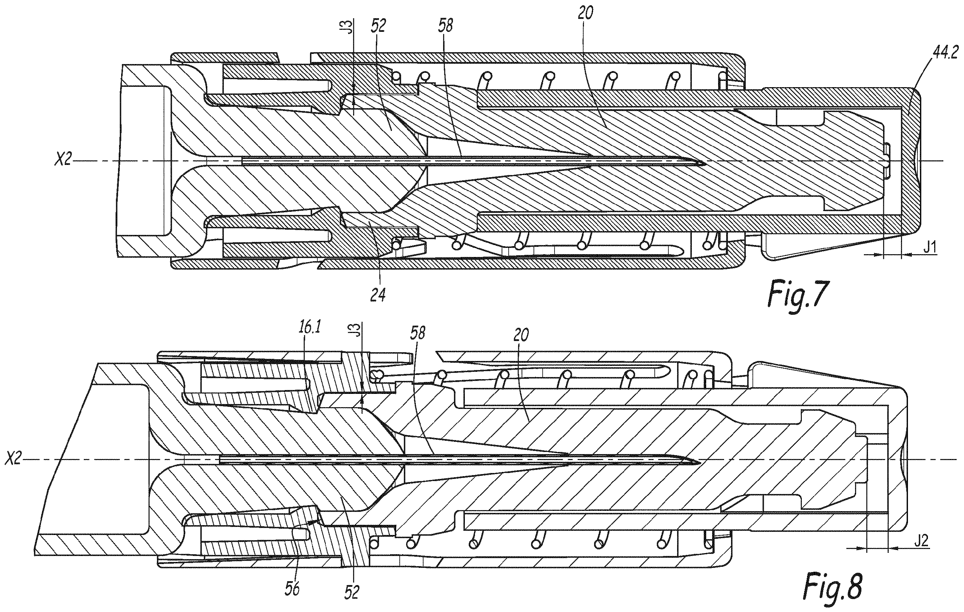

Dès que l'on relâche la pression sur le dispositif 2, le ressort 30 se détend légèrement ce qui fait avancer le fourreau 40 de quelques millimètres. Cela est particulièrement visible en regardant la différence de jeu axial entre l'extrémité distale du protège-aiguille 20 et la paroi d'extrémité 44.2 du tube interne 42. Effectivement, le jeu axial J2 à la figure 8 est supérieur au jeu axial J1 à la figure 7. Ce léger déplacement permet d'éloigner les surfaces fonctionnelles de la nervure 44.1 de la surface extérieure du protège-aiguille 20, ce qui limite le risque d'accrochage du protège-aiguille avec le tube 44 lorsque le tube 44 est tourné pour être détaché, et donc le risque de carottage.

Avant d'effectuer une piqûre, l'utilisateur tourne le tube interne 44 autour de son axe, de façon à rompre les pontets 46. Par la suite, l'utilisateur peut détacher le tube interne 44 par un mouvement de retrait axial et enlève en même temps le protège-aiguille 20. On comprend donc que le retrait du protège-aiguille 20 s'effectue en deux temps. Ceci était en partie l'objet de la demande WO 2016/120185 A1. On décompose ainsi les efforts liés au retrait du protège-aiguille. A l'inverse, dans l'art antérieur, les pontets étaient cassés en tirant sur la partie détachable, si bien que les efforts axiaux nécessaires à la rupture des pontets s'additionnaient à ceux nécessaires au déplacement du protège-aiguille, et que l'effort de traction était donc relativement élevé.

Avantageusement, le bord chanfreiné 42.20 formé à l'extrémité distale 42.2 du tube externe 42 s'oppose à la réinsertion du protège-aiguille 20 à l'intérieur du tube 42 et bute notamment contre le bord franc 22.2 de la collerette. On dissuade ainsi l'utilisateur de remettre l'ensemble comprenant le tube 44 et le protège-aiguille 20 à l'intérieur du tube 42 après l'injection, ce qui supprime le risque de piqure après injection.

En revanche, du fait de son inclinaison évasée vers l'intérieur du tube 42, le bord 42.20 ne s'oppose pas au déplacement du protège-aiguille 20 à l'extérieur du tube 42, par glissement de la pente 22.1 contre le bord 42.2.

Les étapes pour réaliser l'injection du principe actif dans le corps d'un patient, c'est-à-dire la méthode d'utilisation de la seringue, sont identiques à celles divulguées dans WO 2016/120185 A1 et WO 2016/207196 A1. C'est pourquoi, par souci de concision, ces étapes ne sont pas reprises dans le présent document.

En variante non représentée, le dispositif comporte une inscription (marquage), par exemple un pictogramme ou une flèche, pour indiquer à l'utilisateur la manœuvre à effectuer pour retirer le protège-aiguille 20. Par exemple, ce marquage peut être une flèche, à double sens ou à sens unique, indiquant un mouvement de rotation. Une autre flèche peut être prévue pour indiquer le mouvement de traction.

Selon une autre variante non représentée, la partie visible du tube interne 44, c'est-à-dire la partie du tube 44 disposée à l'extérieur du tube 42, comprend au moins une, de préférence plusieurs ouvertures radiales, favorisant le passage du gaz de stérilisation à l'oxyde d'éthylène (ETO).

Les caractéristiques du mode de réalisation et des variantes peuvent être combinées entre elles pour générer de nouveaux modes de réalisation de l'invention.

## Revendications

1. Dispositif (2) de protection d'aiguille pour une seringue pré-remplie à aiguille collée (1), le dispositif comprenant :
- un bloqueur (10), comprenant des moyens (16) de fixation sur un corps de seringue (50),
- un protège-aiguille (20), à l'intérieur duquel l'aiguille (58) peut être enfoncée,
- un système de sécurité après usage (30, 40), comprenant un fourreau (40) définissant un axe central selon lequel il est mobile et un moyen (30) pour charger élastiquement le fourreau dans une position avancée où il est apte à recouvrir l'aiguille, le fourreau comprenant :
o un tube externe (42), agencé de façon à coopérer avec le bloqueur (10), et
o un tube coaxial interne (44), disposé coaxialement à l'intérieur du tube externe (42) et dépassant axialement, côté distal, par rapport au tube externe,
**caractérisé en ce que** le tube externe (42) et le tube coaxial interne (44) sont fabriqués en une seule pièce par injection plastique, **en ce que** le tube externe (42) et le tube interne (44) sont attachés l'un à l'autre par des pontets sécables (46), et **en ce que** le tube interne (44) est détachable du tube externe et lié axialement en translation avec le protège-aiguille (20).

2. Dispositif selon la revendication 1, **caractérisé en ce que** le protège-aiguille (20) comprend une collerette (22) interposée axialement entre le bloqueur (10) et le tube interne (44) du fourreau.

3. Dispositif selon la revendication 1, **caractérisé en ce que** les pontets sécables (46) sont formés à une extrémité axiale (42.2) du tube externe.

4. Dispositif selon l'une des revendications précédentes, **caractérisé en ce que** le tube interne (44) comprend une extrémité fermée.

5. Dispositif selon l'une des revendications précédentes, **caractérisé en ce que** le tube interne (44) présente un diamètre extérieur (D44) minimum strictement inférieur à un diamètre extérieur (D14) d'une partie d'extrémité distale (14) du bloqueur (10).

6. Dispositif selon l'une des revendications précédentes, **caractérisé en ce que** le tube interne (44) est opaque et le tube externe (42) est transparent.

7. Dispositif selon l'une des revendications précédentes, **caractérisé en ce que** le tube interne (44) et le tube externe (42) présentent un état de surface différent.

8. Dispositif selon l'une des revendications précédentes, **caractérisé en ce que** le tube interne comprend des ailettes de préhension (48).

9. Seringue pré-remplie à aiguille collée (1) comprenant un dispositif (2) selon l'une des revendications précédentes.

## Patentansprüche

1. Nadelschutzvorrichtung (2) für eine vorgefüllte Spritze mit abgesteckter Nadel (1), wobei die Vorrichtung umfasst:
- eine Arretierung (10), die Befestigungsmittel (16) auf einem Spritzenkörper (50) umfasst,
- einen Nadelschutz (20), in den die Nadel (58) eingeführt sein kann,
- ein Sicherheitssystem nach dem Gebrauch (30, 40), das eine Hülle (40) umfasst, die eine zentrale Achse definiert, der gemäß es beweglich ist und ein Mittel (30), um die Hülle in einer vorgeschobenen Position elastisch zu laden, in der sie imstande ist, die Nadel zu bedecken, wobei die Hülle umfasst:
o ein äußeres Rohr (42), das derart eingerichtet ist, dass es mit der Arretierung (10) zusammenwirkt, und
o ein inneres koaxiales Rohr (44), das koaxial im Inneren des äußeren Rohrs (42) angeordnet ist und distalseitig im Verhältnis zum äußeren Rohr übersteht,
**dadurch gekennzeichnet, dass** das äußere Rohr (42) und das koaxiale innere Rohr (44) aus einem einzigen Teil durch plastische Injektion hergestellt sind und dass das äußere Rohr (42) und das innere Rohr (44) durch trennbare Bügel (46) aneinander befestigt sind und dass das innere Rohr (44) vom äußeren Rohr lösbar ist und mit dem Nadelschutz (20) axial translatorisch verbunden ist.

2. Vorrichtung nach Anspruch 1, **dadurch gekennzeichnet, dass** der Nadelschutz (20) einen Flansch (22) umfasst, der zwischen der Arretierung (10) und dem inneren Rohr (44) der Hülle axial zwischengestellt ist.

3. Vorrichtung nach Anspruch 1, **dadurch gekennzeichnet, dass** die trennbaren Bügel (46) an einem axialen Ende (42.2) des äußeren Rohrs gebildet sind.

4. Vorrichtung nach einem der vorangehenden Ansprüche, **dadurch gekennzeichnet, dass** das innere Rohr (44) ein geschlossenes Ende umfasst.

5. Vorrichtung nach einem der vorangehenden Ansprüche, **dadurch gekennzeichnet, dass** das innere Rohr (44) einen Mindest-Außendurchmesser (D44) aufweist, der strikt kleiner als ein Außendurchmesser (D14) eines distalen Endteils (14) der Arretierung (10) ist.

6. Vorrichtung nach einem der vorangehenden Ansprüche, **dadurch gekennzeichnet, dass** das innere Rohr (44) undurchsichtig ist und das äußere Rohr (42) durchsichtig ist.

7. Vorrichtung nach einem der vorangehenden Ansprüche, **dadurch gekennzeichnet, dass** das innere Rohr (44) und das äußere Rohr (42) einen unterschiedlichen Oberflächenzustand aufweisen.

8. Vorrichtung nach einem der vorangehenden Ansprüche, **dadurch gekennzeichnet, dass** das innere Rohr Greifflügel (48) umfasst.

9. Vorgefüllte Spritze mit abgesteckter Nadel (1), umfassend eine Vorrichtung (2) nach einem der vorangehenden Ansprüche.

## Claims

1. A needle protection device (2) for a pre-filled syringe with staked needle (1), the device comprising:
- a blocking mechanism (10), comprising means (16) for fastening to a syringe body (50),
- a needle shield (20), into which the needle (58) can be driven,
- an after-use safety system (30, 40), comprising a sleeve (40) defining a central axis along which it is movable and means (30) for elastically loading the sleeve in a forward direction in which it is able to cover the needle, the sleeve comprizing:
o an outer tube (42), which is arranged so as to cooperate with the blocking mechanism (10), and
o a coaxial inner tube (44), that is arranged coaxially inside the outer tube (42) and that protrudes axially, on the distal side, relative to the outer tube (42),
**characterized in that** the outer tube (42) and the inner tube (44) are manufactured in one piece by injection molding, **in that** the outer tube (42) and the inner tube (44) are attached to one another by frangible bridges (46) and **in that** the inner tube (44) is detachable from the outer tube and axially connected in translation with the needle shield (20).

2. The device according to claim 1, **characterized in that** the needle shield (20) comprises a collar (22) inserted axially between the blocking mechanism (10) and the inner tube (44) of the sleeve.

3. The device according to claim 1, **characterized in that** the frangible bridges (46) are formed at a distal end (42.2) of the outer tube.

4. The device according to one of the preceding claims, **characterized in that** the inner tube (44) comprises a closed end.

5. The device according to one of the preceding claims, **characterized in that** the inner tube (44) has a minimum outer diameter (D44) strictly smaller than an outer diameter (D14) of a distal end part (14) of the blocking mechanism (10).

6. The device according to one of the preceding claims, **characterized in that** the inner tube (44) is opaque and the outer tube (42) is transparent.

7. The device according to one of the preceding claims, **characterized in that** the inner tube (44) and the outer tube (42) have different surface states.

8. The device according to one of the preceding claims, **characterized in that** the inner tube comprises gripping fins (48).

9. A prefilled syringe with staked needle (1) comprising a device (2) according to one of the preceding claims.
